## Europäisches Patentamt
## European Patent Office
## Office européen des brevets

(19)

(11) Publication number: **0 119 664**
**B1**

(12)

# EUROPEAN PATENT SPECIFICATION

(45) Date of publication of patent specification: 07.06.89

(51) Int. Cl.⁴: **A 61 B 6/02, G 06 F 15/42**

(21) Application number: 84200386.5

(22) Date of filing: 16.03.84

(54) Tomography data acquisition system.

(30) Priority: 17.03.83 US 476102

(43) Date of publication of application:
26.09.84 Bulletin 84/39

(45) Publication of the grant of the patent:
07.06.89 Bulletin 89/23

(84) Designated Contracting States:
DE FR NL

(56) References cited:
EP-A-0 050 510
US-A-4 206 359
US-A-4 222 104
US-A-4 259 721

TOPICS IN APPLIED PHYSICS, vol. 32, publ.
1979, by Springer-Verlag, New York, G.T.
HERMAN "Image reconstruction from
projections", pp. 1-7

(73) Proprietor: ANALOGIC CORPORATION
8 Centennial Drive Centennial Park
Peabody Massachusetts 01961 (US)

(72) Inventor: Dobbs, John McG.
218 Sagamore Street
So. Hamilton Massachusetts 01982 (US)
Inventor: Gordon, Bernard M.
136 Hesperus Avenue
Magnolia Massachusetts 01930 (US)

(74) Representative: Smulders, Theodorus A.H.J., Ir.
et al
Vereenigde Octrooibureaux Nieuwe Parklaan
107
NL-2587 BP 's-Gravenhage (NL)

**Description**

This invention relates to a data acquisition system for use in a tomography system which includes a radiation source and a single radiation detector, which data acquisition system comprises means operative to provide digitized representations of the output signals of said detector; and means operative to provide a sampling rate and/or resolution of the digitized representations of the detector signals.

The invention relates also to a data acquisition system for use in a tomography system which includes a radiation source and a plurality of radiation detector, which data acquisition system comprises means operative to provide digitized representations of detector signals corresponding to respective radiation paths through a tomography subject; and means operative to provide a sampling rate and/or resolution of the digitized signal representations of the detector signals.

In a tomography system, a detector, which usually comprises an array of detector elements, provides signals in response to radiation received along different radiation paths through a subject, and these signals are processed to reconstruct a tomographic image of the subject. Such a tomography system is known from EP—A1—0050510. In the design of systems for the acquisition of data from the radiation detector, it is assumed that the information content for each radiation path through a subject is the same. It has been found that the information content is not the same for all radiation paths and that the information content is less for paths outward of a central path through the center of a subject. The present invention makes use of such recognition to provide a tomography data acquisition system in which the data channels are implemented in accordance with the respective information content for the associated radiation paths, with resultant reduction in the cost and quantity of signal processing apparatus needed for outward radiation paths.

To this end the data acquisition system according to the invention is characterized in that this sampling rate and/or resolution is a function of the position of the detected rays relative to a reference line from the radiation source through the center of a tomography subject.

The invention also relates to a data acquisition system for use in a tomography system which includes a radiation source and a detector array disposed along a segment of an arc in spaced relation to the radiation source. This data acquisition system being characterized in that the data acquisition system comprises first signal processor means coupled to the detectors of the detector array within a central segment centered about a center line between the radiation source and the array; at least second signal processor means coupled to the detectors of the detector array within segments outward of each side of the central segment; the second signal processor means having a sampling rate and/or resolution less than the first signal processor.

In brief, the invention provides a tomography data acquisition system in which the signal processing of signals from a radiation detector is a function of the angular position of the detector relative to a center path from the radiation source through the center of a tomography subject. The information content of detector signals derived from radiation paths progressively outward from the center path is progressively less than the information content of detector signal derived from the central radiation path. The frequency spectrum of detector signals derived from the outward paths is lower, and the sampling rates of these detector signals can be progressively lower without loss of information. The resolution of the detector signals can also be progressively lower for signals corresponding to the progressively outward radiation paths, by reason of the smaller data contribution of the outward paths occasioned by less target information existing along outward radiation paths through a subject. Either the sampling rate or the resolution or both can be varied in accordance with the position of the detector. The angular resolution of detectors at outward path positions can also be less than for the central paths. By virtue of the invention, the signal processors for outward radiation paths can be slower and of lower resolution and thus of less cost. Additionally, less signal processing apparatus need be employed for outward radiation paths.

Although the sampling rate is less for each successive outward detector position, separate processing of each detector signal at a respective rate is not usually necessary. It is sufficient for practical implementation of the invention to separately process the detector signals from the outermost radiation paths where an appreciable decrease in sampling rate is achievable. For a detector at a distance 7/8 of the maximum distance from the center line, the sampling rate need only be about half the rate needed at the center line position.

The invention is useful in various types of tomography systems including fan beam, inverse fan beam and parallel beam systems.

Description of the drawings

The invention will be more fully understood from the following detailed description in conjunction with the accompanying drawings, in which:

Fig. 1 is a diagrammatic representation of a fan beam tomography system in accordance with the invention;

Fig. 2 is a diagrammatic representation of an alternative embodiment of the invention in a fan beam system;

Fig. 3 is a block diagram of a typical signal processor useful in the embodiments of Figs. 1 and 2;

Fig. 4 is a diagrammatic representation of an inverse fan beam tomography system in accordance with the invention; and

Fig. 5 is a diagrammatic representation of a parallel beam tomography system in accordance with the invention.

## Detailed description of the invention

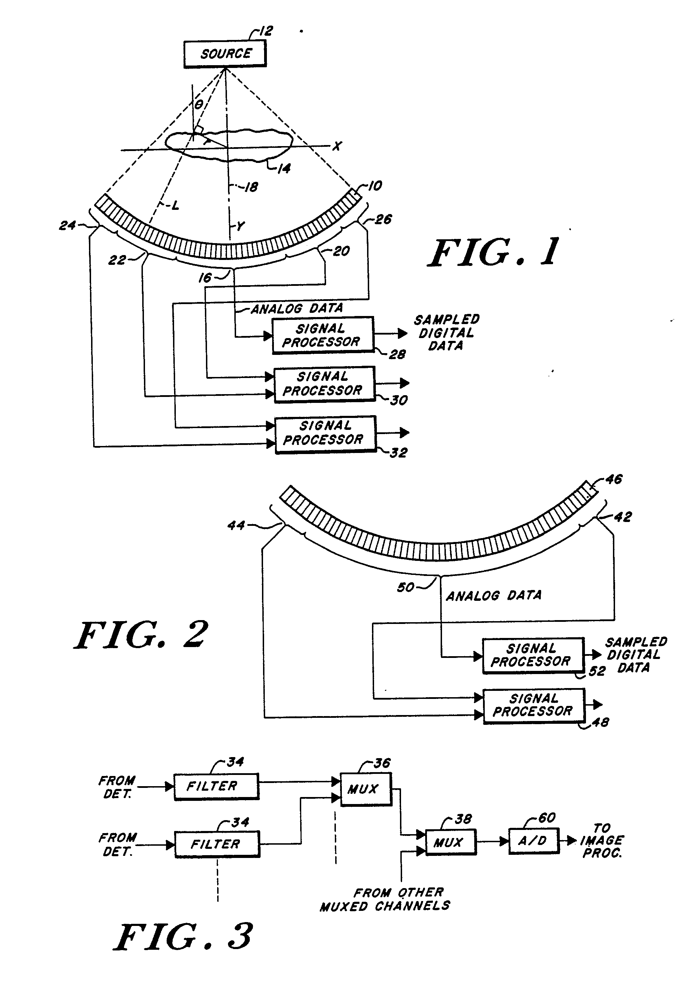

Referring to Fig. 1, a fan beam tomography system is shown having a radiation detector 10 composed of an array of detector elements disposed along a segment of an arc and in spaced relation with a radiation source 12. The X-ray source and detector array are disposed on a rotating assembly and are continuously rotatable as a unit about a subject 14 which is in stationary position between the source and the detector array and through which radiation from the source is projected for detection by the detectors of the array. The detector array includes a central segment 16 roughly centered about center line 18, segments 20 and 22 outward of each side of the central segment 16, and outermost segments 24 and 26 at respective ends of the array. The detector signals from the central segment 16 of the detector array are applied to a signal processor 28. The detector signals from the segments 20 and 22 are applied to a signal processor 30, and the detector signals from the outermost segments 24 and 26 are applied to a signal processor 32. The X-ray source is operated continuously, causing the detectors to produce continuous signals, which are processed by the respective signal processors which provide sampled digital data for image reconstruction and display of a tomographic image.

Two coordinate systems are illustrated in Fig. 1. A rectangular coordinate system is centered about the axis of rotation and has mutually orthogonal axes x and y. The radiation paths, such as path L, are specified within a polar coordinate system by the radiation distance r from the origin, and the angular orientation θ between the path L and the y axis. The relationship between the two coordinate systems is defined by the Radon transform, as is well understood in the art of computerized tomography. See, for example, *Image Reconstruction From Projections*, Gabor T. Herman, Academic Press, 1980. Note that sampling in time for the moving detector array corresponds to sampling in angle (θ) of the subject's Radon transform.

The signal processors are typically implemented as shown in Fig. 3. Each detector signal is applied to a respective filter 34, the filter outputs from a group of detectors being applied to a multiplexer 36. The output of the multiplexer 36 is applied to a second multiplexer 38 which also receives multiplexed signals from other groups of detectors. The second multiplexer 38 is employed to achieve a fan-in of multiple detector detector signals which is greater than may be possible with a single multiplexer. The output from multiplexer 38 is applied to an analog-to-digital converter 40, the output of which is the sampled digital data which is applied to an image processor reconstruction of a tomography image for further processing and/or display.

In accordance with the invention, the sampling rate of signal processor 30 is less than the sampling rate of signal processor 28, and the sampling rate of signal processor 32 is less than that of signal processor 30. The sampling rate of each signal processor is determined in accordance with known criteria to provide digital data which correctly represents the spectral content of the input analog signals. Since the spectrum of the signals provided by the detectors in segments 20 and 22 is lower than the signal spectrum derived from central segment 16, the sampling rate of signal processor 30 is less than the rate of signal processor 28 and yet is sufficient to properly encode the input signals. The sampling rate of signal processor 32 is still lower than that of signal processor 30 because of the still lower spectral content of the signals derived from the outer segments 24 and 26. The lower sampling rates can be accomplished with slower signal processors with consequent reduced cost for such processors.

The range of data contributed by the radiation paths angularly outward from the center line is less than the range of data contributed by the centrally disposed radiation paths, and thus less resolution is needed in encoding the detector signals from the outer positions. The resolution of the analog-to-digital conversion can, therefore, be less in signal processor 30 and still less in signal processor 32, in comparison to the conversion resolution of signal processor 28. The lower analog-to-digital conversion resolution also allows use of converters of slower speed and less cost.

The sampling rate and resolution can both be lower in signal processor 30 in relation to the sampling rate and resolution of signal processor 28. Alternatively, either the sampling rate or the resolution can be lower than that of signal processor 28. Similarly, either the sampling rate or resolution, or both, of signal processor 32 can be lower than the corresponding parameters of signal processor 30.

Since the information content derived from the radiation paths impinging on the outer segments of the detector array are less, the angular resolution of the detectors disposed in these outer segments of the array can be less. Thus, a lesser number of detectors can be provided in segments 20 and 22, and a still lesser number of detectors can be provided in segments 24 and 26. This reduction in the angular resolution of the detectors along the array can be employed in conjunction with the reduced sampling rate and reduced converter resolution discussed above. As a result, less apparatus can be employed in implementation of the signal processing for the outward radiation paths.

An alternative embodiment is shown in Fig. 2 in which the detectors of the outermost segments 42 and 44 of the detector array 46 are coupled to a signal processor 48, while the detectors of the intermediate segment 50 are coupled to a signal processor 52. An appreciable decrease in sampling rate is achievable at the outermost end portions 42 and 44 of the detector array, and thus the signal processor 48 operates at a sampling rate substantially less than the sampling rate of signal processor 52. The resolution of the analog-to-digital conversion in signal processor 48 can also be less than the resolution in signal processor 52. As discussed above, the signal processor 48 can provide either lower sampling rate or lower resolution or both in comparison to that of signal processor 52.

The relative optimal sampling frequency as a function of distance from the center line is indicated in the following table.

| Distance from center line | Sampling frequency |
|---|---|
| 0 | 1.00 |
| 1/2 | .87 |
| 3/4 | .66 |
| 7/8 | .48 |
| 15/16 | .35 |

The detectors at outward distances of 7/8 of the maximum distance need only be sampled at about half the rate of the detector at the center line. Thus, sampling can be accomplished with a 13 bit converter rather than a 14 bit converter as typically required by conventional systems.

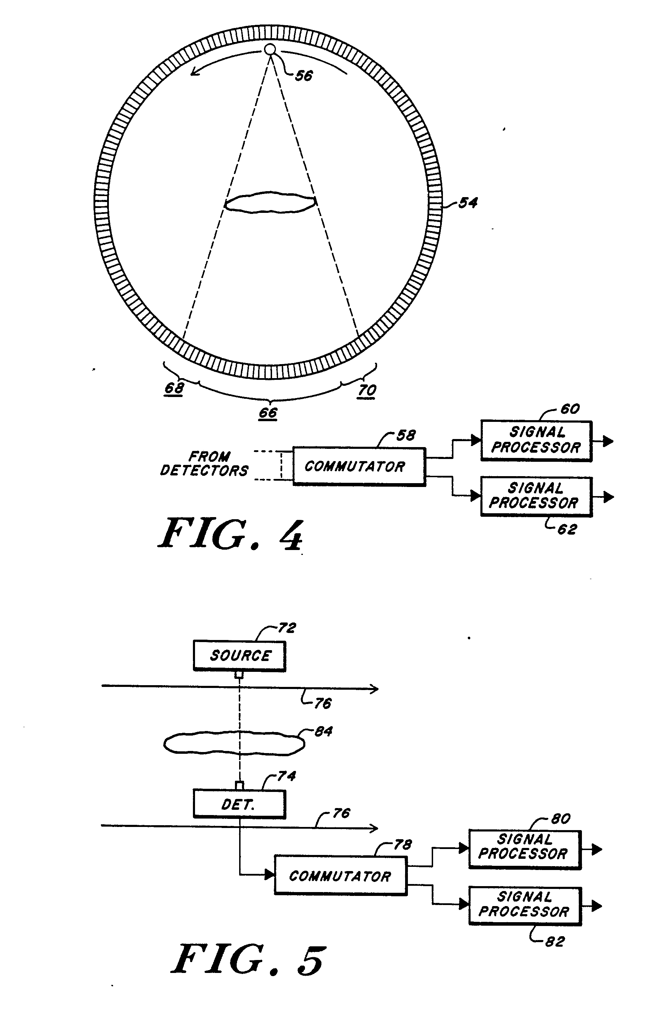

The above discussion describes the invention embodied in a fan beam tomography system. The invention as embodied in an inverse fan beam tomography system is depicted in Fig. 4. Here an array of stationary detectors 54 is disposed about the circumference of a circle within which a rotary assembly carrying an X-ray source 56 is supported. At any rotational position of the source, a plurality of detectors is illuminated, and during rotation of the source, the detectors composing the active group are continuously changing as each detector comes within the sector of energization and another detector falls outside of the energized sector. The detector signals are applied to a commutator 58 which provides output signals to respective signal processors 60 and 62. The commutator 58 is operative to couple the illuminated group of detectors 54 to the signal processor in accordance with rotation of the X-ray source in known manner. In accordance with the invention, the commutator is also operative to couple the detectors to the respective signal processors 60 and 62 in accordance with the position of those detectors within the active sector. Thus, for the position illustrated in Fig. 4, the centrally disposed detectors 66 are coupled by the commutator 58 to the signal processor 60, while the outwardly disposed detectors 68 and 70 are coupled by the commutator to the signal processor 62. The signal processor 62 for the outer detectors has a sampling rate and/or lower conversion resolution than signal processor 60.

The invention as embodied in a parallel beam tomography system is shown in Fig. 5 in which an X-ray source 72 and single detector 74 are spaced from each other and movable together along a translatory path illustrated by arrows 76. The source usually includes a collimator to provide a narrow radiation beam for impingement on the single detector. The detector signal is applied to a commutator 78 which provides signals to first and second signal processors 80 and 82. The commutator provides signals to one or the other of the signal processors as a function of the position of the detector in relation to the subject 84 under examination. For detector positions outward of a center line through the subject, the commutator 78 is operative to provide the detector signals to the signal processor 82 having lower sampling rate and/or lower resolution. For detector positions within a predetermined central region disposed about the center line, the commutator provides signals to the signal processor 80 which operates at the higher sampling rate and/or resolution. The translational speed of the detector can also be varied as a function of the position of the detector in relation to the center line.

In an alternative implementation of the parallel beam system, the commutator 78 can be eliminated, and a single signal processor can be employed having a variable sampling capability to provide a sampling rate in accordance with the position of the detector relative to the subject. The signal processor can also have a variable resolution in accordance with detector position.

It will be appreciated that a tomography data acquisition system can be constructed in accordance with the principles of the invention in a more cost effective manner. The analog-to-digital converters for processing of signals from the outward radiation paths can be slower and/or of lower resolution and therefore less expensive. Fewer converters can also be employed for the outward paths with resultant hardware saving.

## Claims

1. Data acquisition system for use in a tomography system which includes a radiation source and a single radiation detector, which data acquisition system comprises means operative to provide digitized representations of the output signals of said detector; and means operative to provide a sampling rate and/or resolution of the digitized representations of the detector signals, characterized in that this sampling rate and/or resolution are/is a function of the position of the detected rays relative to a reference line from the radiation source through the center of a tomography subject.

2. Data acquisition system for use in a tomography system which includes a radiation source and a plurality of radiation detector, which data acquisition system comprises means operative to provide digitized representations of detector signals corresponding to respective radiation paths through a tomography subject; and means operative to provide a sampling rate and/or resolution of the digitized signal representations of the detector signals, characterized in that this sampling rate and/or this resolution are/is a function of the position of the detected rays relative to a reference line from the radiation source through the center of a tomography subject.

3. Data acquisition system for use in a tomography system which includes a radiation source and a detector array disposed along a segment of an arc in spaced relation to the radiation source, characterized in that the data acquisition system comprises first signal processor means coupled to the detectors of the detector array within a central segment centered about a center line between the radiation source and the array; at least second signal processor means coupled to the detectors of the detector array within segments outward of each side of the central segment; the second signal processor means having a sampling rate and/or resolution less than the first signal processor.

4. The data acquisition system of claim 3 characterized in that the first and second signal processor means each include analog-to-digital converter means providing sampled digitial data representative of analog data derived from the detectors of the detector array; the analog-to-digital converter means of the second signal processor means having a sampling rate less than the sampling rate of the analog-to-digital converter means of the first signal processor means.

5. The data acquisition system of claim 3, characterized in that the first and second signal processor means each include analog-to-digital converter means providing sampled digital data representative of analog data derived from the detectors of the detector array; the analog-to-digital converter means of the second signal processor means having a conversion resolution less than the resolution of the analog-to-digital converter means of the first signal processor means.

6. The data acquisition system of claim 3, characterized in that the first and second signal processor means each include analog-to-digital converter means providing sampled digital data representative of analog data derived from the detectors of the detector array; the analog-to-digital converter means of the second signal processor means having a sampling rate and a conversion resolution less than those of the analog-to-digital converter means of the first signal processor means.

7. The data acquisition system of claim 3, characterized in that the angular resolution of detectors of said array is less for detectors at the outward segments.

## Patentansprüche

1. Datenerfassungssystem zum Einsatz in einem Tomographiesystem, das eine Strahlungsquelle und einen einzigen Strahlendetektor aufweist, wobei das Datenerfassungssystem Mittel, die digitalisierte Darstellungen der Ausgangssignale des Detektors erzeugen, und Mittel aufweist, welche eine Abtastrate und/oder Auflösung der digitalisierten Darstellungen der Detektorsignale festlegen, dadurch gekennzeichnet, daß diese Abtastrate und/oder Auflösung eine Funktion der Position der erfaßten Strahlen relativ zu einer Bezugslinie von der Strahlungsquelle durch das Zentrum eines Tomographiesubjekts sind bzw. ist.

2. Datenerfassungssystem zum Einsatz in einem Tomographiesystem, das eine Strahlungsquelle und eine Mehrzahl von Strahlungsdetektoren aufweist, wobei das Datenerfassungssystem Mittel, die digitalisierte Darstellungen von Detektorsignalen entsprechend zugehöriger Strahlungswege durch ein Tomographiesubjekt erzeugen, und Mittel aufweist, welche eine Abtastrate und/oder Auflösung der digitalisierten Signaldarstellungen der Detektorsignale festlegen, dadurch gekennzeichnet, daß diese Abtastrate und/oder Auflösung eine Funktion der Position der erfaßten Strahlen relativ zu einer Bezugslinie von der Strahlungsquelle durch das Zentrum eines Tomographiesubjekts sind bzw. ist.

3. Datenerfassungssystem zum Einsatz in einem Tomographiesystem, das eine Strahlungsquelle und eine Detektoranordnung aufweist, die längs eines Bogensegments mit Abstand von der Stahlungsquelle angeordnet ist, dadurch gekennzeichnet, daß das Datenerfassungssystem erste Signalverarbeitungsmittel, die mit den Detektoren innerhalb eines mittigen, bezüglich einer Mittellinie zwischen der Strahlungsquelle und der Detektoranordnung zentrierten Mittelsegments der Detektoranordnung verbunden sind, und mindestens zweite Signalverarbeitungsmittel aufweist, die mit den Detektoren innerhalb von Segmenten beiderseits des mittigen Segments der Detektoranordnung verbunden sind, wobei die Abtastrate und/oder Auflösung der zweiten Signalverarbeitungsmittel kleiner als bei den ersten Datenverarbeitungsmitteln sind bzw. ist.

4. Datenerfassungssystem nach Anspruch 3, dadurch gekennzeichnet, daß die ersten und zweiten Signalverarbeitungsmittel jeweils einen Analog-Digital-Wandler aufweisen, welcher von den Detektoren der Detektoranordnung abgeleitete Analogdaten darstellende abgetastete Digitaldaten erzeugt, wobei die Analog-Digital-Wandler der zweiten Signalverarbeitungsmittel eine kleiner Abtastrate als die Analog-Digital-Wandler der ersten Signalverarbeitungsmittel aufweisen.

5. Datenerfassungssystem nach Anspruch 3, dadurch gekennzeichnet, daß die ersten und zweiten Signalverarbeitungsmittel jeweils Analog-Digital-Wandler aufweisen, die von den Detektoren der Detektoranordnung abgeleitete Analogdaten darstellende abgetastete Digitaldaten erzeugen, wobei die Analog-Digital-Wandler der zweiten Signalverarbeitungsmittel eine kleinere Umwandlungsauflösung als die Analog-Digital-Wandler der ersten Signalverarbeitungsmittel aufweisen.

6. Datenerfassungssystem nach Anspruch 3, dadurch gekennzeichnet, daß die ersten und zweiten Signalverarbeitungsmittel jeweils Analog-Digital-Wandler aufweisen, welche von den Detektoren der Detektoranordnung abgeleitete Analogdaten darstellende abgetastete Digitaldaten erzeugen, wobei die Analog-Digital-Wandler der zweiten Signalverarbeitungsmittel eine kleinere Abtastrate und Umwandlungsauflösung als die Analog-Digital-Wandler der ersten Signalverarbeitungsmittel aufweisen.

7. Datenerfassungssystem nach Anspruch 3, dadurch gekennzeichnet, daß die Winkelauflösung der Detektoren der Anordnung bei Detektoren der äußeren Segmente kleiner ist.

**Revendications**

1. Système de saisie de données destiné à être utilisé dans un système de tomographie qui comporte une source de rayonnement et un seul détecteur de rayonnement, ce système de saisie de données comportant un dispositif ayant pour fonction de produire des représentations numérisées des signaux de sortie dudit détecteur; et un dispositif ayant pour fonction d'établir une fréquence d'échantillonnage et/ou une résolution des représentations numérisées des signaux du détecteur, caractérisé en ce que cette fréquence d'échantillonnage et/ou résolution sont/est une fonction de la position des rayons détectés par rapport à une ligne de référence depuis la source de rayonnement, passant par le centre d'un sujet de tomogrpahie.

2. Système de saisie de données destiné à être utilisé dans un système de tomographie qui comporte une source de rayonnement et plusieurs détecteurs de rayonnement, ce système de saisie de données comportant un dispositif ayant pour fonction de produire des représentations numérisées des signaux des détecteurs correspondant à des trajets de rayonnements respectifs passant par un sujet de tomographie; et un dispositif ayant pour fonction d'établir une fréquence d'échantillonnage et/ou une résolution des représentations de signaux numérisés des signaux des détecteurs, caractérisé en ce que cette fréquence d'échantillonnage et/ou cette résolution sont/est une fonction de la position des rayons détectés par rapport à une ligne de référence partant de la source de rayonnement et passant par le centre d'un sujet de tomographie.

3. Système de saisie de données destiné à être utilisé dans un système de tomographie qui comporte une source de rayonnement et un réseau de détecteurs disposé le long d'un segment d'un arc à distance de la source de rayonnement, caractérisé en ce que le système de saisie de données comporte un premier dispositif processeur de signaux couplé avec les détecteurs du réseau de détecteurs dans un segment central centré autour d'une ligne centrale entre la source de rayonnement et le réseau; et au moins un second dispositif processeur de signaux couplé avec les détecteurs du réseau de détecteurs dans des segments extérieurs de chaque côté du segment central; le second dispositif processeur de signaux ayant une fréquence d'échantillonnage et/ou une résolution inférieures à celles du premier processeur de signaux.

4. Système de saisie de données selon la revendication 3, caractérisé en ce que le premier et la second dispositifs processeurs de signaux comportant chacun un dispositif convertisseur analogique-numérique produisant des données numériques échantillonnées représentant des données analogiques dérivées des détecteurs du réseau de détecteurs; le dispositif convertisseur analogique-numérique du second dispositif processeur de signaux ayant une fréquence d'échantillonnage inférieure à la fréquence d'échantillonnage du dispositif convertisseur analogique-numérique du premier dispositif processeur de signaux.

5. Système de saisie de données selon la revendication 3, caractérisé en ce que le premier et le second dispositifs processeurs de signaux comportent chacun un dispositif convertisseur analogique-numérique produisant des données numériques échantillonnées représentant des données analogiques dérivées des détecteurs du réseau de détecteurs; le dispositif convertisseur analogique-numérique du second dispositif processeur de signaux ayant une résolution de conversion inférieure à la résolution du dispositif convertisseur analogique-numérique du premier dispositif processeur de signaux.

6. Système de saisie de données selon la revendication 3, caractérisé en ce que le premier et le second dispositifs processeurs de signaux comportent chacun un . dispositif convertisseur analogique-numérique produisant des données numériques échantillonées représentant des données analogiques dérivées des détecteurs du réseau de détecteurs; le dispositif convertisseur analogique-numérique du second dispositif processeur de signaux ayant une fréquence d'échantillonnage et une résolution de conversion inférieures à celles du dispositif convertisseur analogique-numérique du premier dispositif processeur de signaux.

7. Système de saisie de données selon la revendication 3, caractérisé en ce que la résolution angulaire des détecteurs dudit réseau est moindre pour des détecteurs aux segments extérieurs.

FIG. 1

FIG. 2

FIG. 3

FROM
DETECTORS

COMMUTATOR ~58

SIGNAL PROCESSOR ~60

SIGNAL PROCESSOR ~62

*FIG. 4*

SOURCE ~72

~76

~84

DET. ~74

~76

COMMUTATOR ~78

SIGNAL PROCESSOR ~80

SIGNAL PROCESSOR ~82

*FIG. 5*

2